# EUROPEAN PATENT APPLICATION

(11) **EP 2 570 421 A1**
(43) Date of publication of application: **20.03.2013**
(21) Application number: 11181760.7
(22) Date of filing: 19.09.2011
(51) Int. Cl.: C07K 1/18, G01N 33/68

(54) **Method to purify and concentrate prions from complex mixtures such as plasma into a target solution**

(71) Applicant: Prionatis AG, 6055 Alpnach Dorf (CH)
(72) Inventor: Ruinatscha, Reto, Dr., 8005 Zürich (CH); Hagen, Alexander, 6025 Neudorf (CH)
(74) Representative: Heubeck, Christian

(57) **Abstract**

The present invention relates to a method for isolating, concentrating and/or purifying amyloids and proteins with the ability to become amyloidogenic as well as to a method for in vitro diagnosis of amyloid diseases.

## Description

### FIELD OF THE INVENTION

The present invention relates to a method for isolating, concentrating and/or purifying amyloids and proteins with the ability to become amyloidogenic.

### BACKGROUND

Amyloids are fibrillar aggregates formed by normally soluble, innocuous proteins or peptides, and are characterized by a cross-β sheet quaternary structure. Diseases featuring amyloids include, but are not limited to, Alzheimer's disease, Diabetes mellitus type 2, Parkinson's disease, Huntington's Disease, and transmissible spongiform encephalopathies. (See, e.g., Chiti & Dobson (2006) Ann. Rev. Biochem. 75: 333-366; Westermark (2005) FEBS J. 272: 5942-5949; Ross & Poirier (2004) Nature Med.10: S10-S17; Dobson (2002) Nature 418: 729-730).

Transmissible spongiform encephalopathies (TSEs) are neurodegenerative diseases. They are largely untreatable and fatal. (See, e.g., Grillberger et al (2009) Biotechnol. J. 4: 186-201). TSEs include bovine spongiform encephalopathy (BSE) in cattle, chronic wasting disease (CWD) in deer and elk, scrapie in sheep and goats, as well as Creutzfeldt-Jakob disease (CJD), Gerstmann-Straussler-Scheinker (GSS) syndrome, fatal familial insomnia (FFI), and Kuru in humans. (See, e.g., Collinge (1999) Lancet 354: 317-323).

TSEs are associated with a conformational conversion of the cellular prion protein PrP^{C} into the pathogenic, β-sheet-rich prion protein PrP^{Sc}. PrP^{Sc} aggregations form highly structured amyloid fibers, which accumulate to form amyloid plaques in affected brains. (See, e.g., Kelly (1996) Curr. Opin. Struct. Biol. 6: 11-17; Pan et al (1993) P. Natl. Acad. Sci. USA 90(23): 10962-10966). Pathological prion forms may have various abbreviations that emphasize associations with infectivity (PrP^{Sc}), disease (e.g. PrP^{TSE} and PrP^{d}), toxicity (PrP^{tox} and PrP^{L}), and relative protease-resistance (PrPres).

TSEs, or prion diseases, can arise spontaneously, e.g. sporadic Creutzfeldt-Jakob disease (sCJD), through genetic predisposition, e.g. familial CJD, or through contaminated instruments, organ and tissue grafts, or oral transmission, e.g. variant CJD (vCJD). (See, e.g., Prusiner (2004) Prion Biology and Diseases, Cold Spring Harbor Lab Press, Woodbury, NY, 2nd Ed.).

Current findings strongly indicate that vCJD may also be transmitted via blood transfusions. (See, e.g., McCutcheon et al (2011) PLoS One 6(8): e23169; Chohan et al (2010) Transfusion 50: 1003-1006; Gillies et al (2009) Vox Sang. 97: 211-218; Ward et al (2009) Vox Sang.97: 207-210; Health Protection Agency (2007) Fourth case of transfusion-associated variant-CJD infection. Health Protection Report 1(3); Health Protection Agency (2006) New case of transfusion-associated variant-CJD. CDR Weekly 16(6); Hewitt PE et al (2006) Vox Sang. 91: 221-230; Wroe et al (2006) Lancet 368: 2061-2067; Llewelyn et al (2004) Lancet 363: 417-421; Peden et al (2004) Lancet 364: 527-529). This risk also exists with blood derived therapeutic formulations, and potentially with all therapeutics and cosmetics which contain components of human or bovine source.

In addition, studies suggest that urine of prion-infected individuals may be infectious before the appearance of clinical signs, i.e. even before pathogenic prions are detectable by current methods in the urine, and that administration of urine-derived products could expose patients to a potential risk of prion transmission. (See, e.g., Dorsselaer et al (2011) PLoS One 6 (3): e17815).

In principle, all amyloid diseases could be infectious under certain conditions. (See, e.g., Sigurdsson (2002) Trends Mol. Med. 8(9): 411-413). For example, in Alzheimer's disease there is emerging evidence that aggregates of amyloid-β may spread from brain region to brain region and from cell to cell in a prion-like fashion. (See, e.g., Holtzman et al (2011) Sci. Transl. Med. 3(77): 77sr1; Kim & Holtzman (2010) Science 330: 918-919). An overview of prions and potential prion-like proteins is provided in the following table (adapted from: Aguzzi (2009) Nature 459(7249): 924-925):

| **Disease** | **Protein** | **Molecular transmissibility** | **Infectious life cycle** |
|---|---|---|---|
| Prion diseases | PrP^{Sc} | Yes | Yes |
| Alzheimer's disease | Amyloid-β | Yes | Not shown |
| Tauopathies | Tau | Yes | Not shown |
| Parkinson's disease | α-Synuclein | Host-to-graft | Not shown |
| AA amyloidosis | Amyloid A | Yes | Possible |
| Huntington's disease | Polyglutamine | Yes | Not shown |

In summary there is a strong need to minimize the potential risk of infection with amyloid diseases, i.e. prion diseases, and at an early stage of these diseases, as this would significantly advance the chances of success of any possible intervention approach.

However, the detection of pathogenic prions, i.e. before the appearance of clinical signs, has proven to be very challenging due to several reasons. Easily accessible body fluids such as plasma contain only very low concentrations of the pathogenic prion protein PrP^{Sc}. The total protein concentration in human plasma is in the range of 60 - 85 mg/ml. In contrast, PrP^{C} concentrations are only around 10 ng/ml, and PrP^{Sc} concentrations of pre-symptomatic CJD patients are assumed to be as low as 30 fg/ml. Such PrP^{Sc} concentrations are by far below the detection limit of established analysis methods such as e.g. Western Blot (detection limit around 30'000 fg/ml) or enzyme-linked immunosorbent assay (ELISA; detection limit around 5'000 fg/ml).

Various methods for the diagnosis of amyloid diseases have been proposed over the past years, whereas on the example of prion diagnostic the following challenges and hurdles have to be addressed:
1. Target protein in protein rich, complex solutions such as e.g. plasma
2. Low concentration of the target proteins below the detection limits of classic immunological analysis methods
3. Pathogenic form (PrP^{Sc}) in much lower concentration than the normal form (PrP^{C})
4. Special efforts required to distinguish the pathogenic from the normal form
5. Overcome practical limitations

As of today only post-mortem tests have been approved by the European health authorities and no in vitro diagnostic test yet ^{[1]}. To specifically address challenges (1), (2), and (5) of above listing several methods are known from the prior art.

Animal bioassays, where prion infectivity is detected itself, take 6 - 7 months to complete and are very costly.

Chromatography with prion specific ligands: the use of known substances immobilized on a solid phase for prion binding, aggregation and/or concentration is also described in the prior art. However, depending on the specific ligand class utilized, practical applications may be limited due to:
(i) High ligand costs, i.e. with antibody-based ligands
(ii) Low affinity of PrP^{Sc} specific ligands, i.e. in solutions containing high concentrations of proteins and/or substances other than prions
(iii) Difficulties in eluting ligand-bound prions for certain analysis methods

The very low concentrations of the TSE agents in body fluids have also been addressed using amplification assays. Protein misfolding cyclic amplification (PMCA) based methods have been shown to detect PrP^{Sc} in plasma from pre-clinical scrapie sheep, whereas quaking-induced conversion (QuIC) was able to discriminate between plasma and serum samples from scrapie-infected and uninfected hamsters in pre-clinical stages. (See, e.g., Rubenstein et al (2010) J. Gen. Virol. 91: 1883-1892; Saa et al (2006) Science 313: 92-94; Orrú et al (2011) mBio 2(3): e00078-11). So far, practical limitations, i.e. with respect to time and handling steps, appear to restrict the use of these methods for prion diagnosis in routine diagnostic or screening applications.

It is therefore one object of the present invention to provide a method for the concentration and/or purification of amyloids and proteins with the ability to become amyloidogenic, and prion proteins in particular, that does not have the disadvantages of the methods known from the prior art. Moreover, it should be possible with the method of the present invention to enable reliable in vitro diagnosis of amyloid diseases, and prion diseases in particular, in an easily applicable manner.

The problem underlying the present invention is solved by providing a method for the selective enrichment or even separation of amyloids and proteins with the ability to become amyloidogenic, in particular prion proteins (e.g. PrP^{c} and/or PrP^{Sc}, and/or PrPres), by immobilized metal ion affinity chromatography (IMAC) from protein-abundant liquids like plasma, and the provision of a flow-through that has been purified from amyloids and proteins with the ability to become amyloidogenic or prions and/or the provision of isolated amyloids and proteins with the ability to become amyloidogenic or prions itself.

Our invention especially offers a solution to the key challenges (1) - complex mixture, challenge (2) - ultra low target protein concentration, and challenge (5) - overcome practical limitations in this aspect, and can be combined with existing, or to be developed, tests, addressing (3) and (4) of above listing. According to a first aspect, the present invention relates to a method for isolating, concentrating and/or purifying amyloids and proteins with the ability to become amyloidogenic, fragments and/or derivatives thereof, comprising a step (a) contacting a sample comprising amyloids and proteins with the ability to become amyloidogenic, fragments and/or derivatives thereof with a material suitable for carrying out immobilized metal affinity chromatography (IMAC), wherein detergents are added to the sample if it was not pretreated with detergents.

This method according to the present invention is characterized by a number of surprising advantages when applied for enrichment and optionally elution.

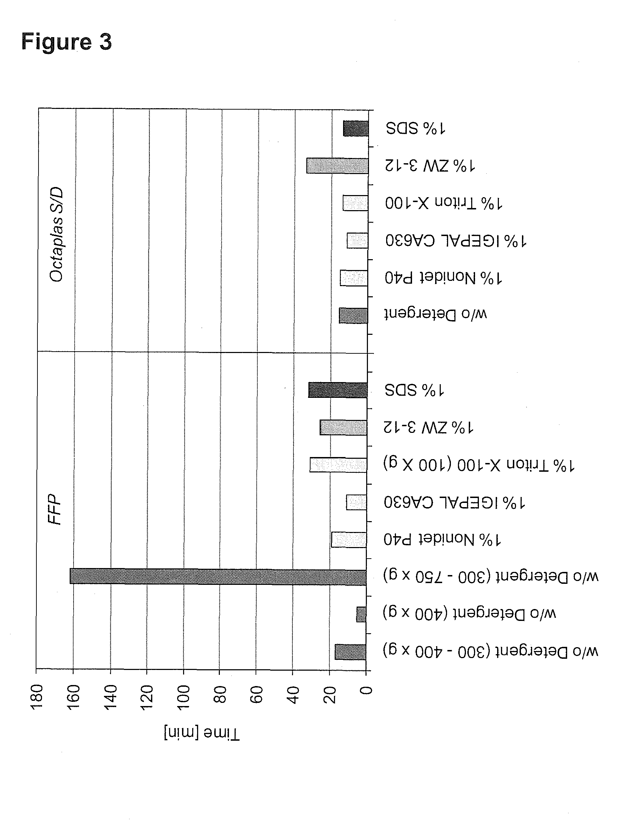

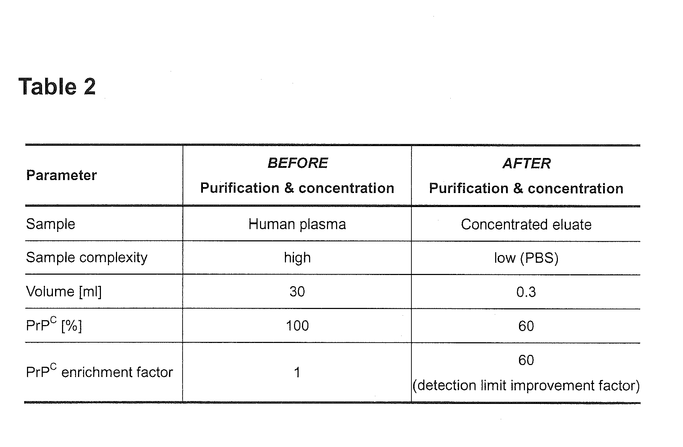

Using the method of the present invention, clogging events are strongly reduced, and too fast flow through times can be minimized, if e.g. prion proteins from plasma are enriched with spin columns at a constant centrifugation force (Table 1, Figure 3).

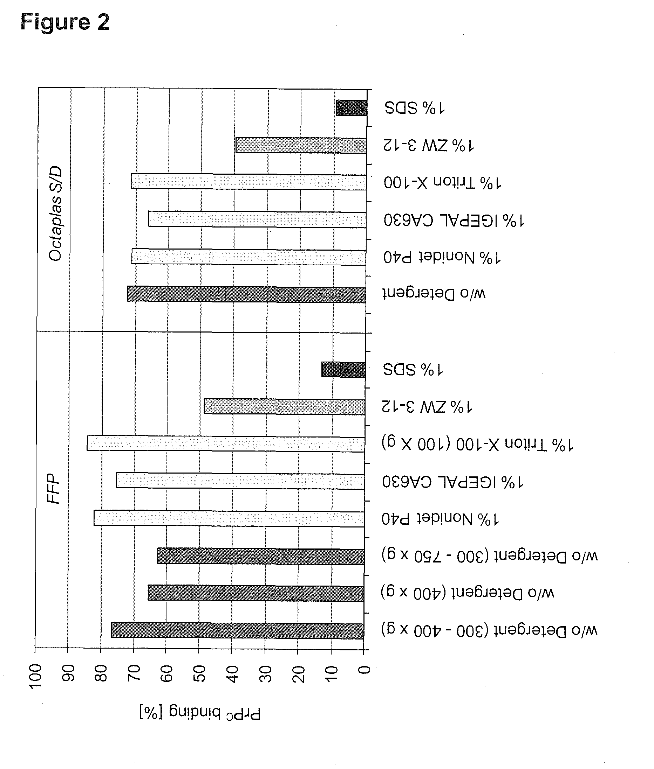

Using the method of the present invention, constant centrifugation forces can be applied to enrich prion proteins from plasma volumes much larger than the IMAC spin column volume, while at the same time ensuring high prion protein binding yields (Table 1, Figure 2).

Technically this is most beneficial, as it allows to work at constant forces without continuous and unpredictable adjustment efforts during the chromatographic process. Only this enables for example the parallel processing of several enrichment processes in spin columns.

Most surprisingly and most important, higher prion protein binding yields are observed with the present invention. This leads to a key advantage over prior art (Table 1, Figure 2).

Using the method of the present invention it is possible to significantly improve the detection limit of low concentrations of amyloids and proteins with the ability to become amyloidogenic. For example, by using the method of the present invention, it was possible to provide a reliable system for the enrichment of prions from 30 ml plasma into a 0.3 ml solution suitable for ELISA analysis, with a final 60-fold improvement of detection limit (Table 2).

The method can easily be carried out by a person skilled in the art who knows standard procedures. It is neither necessary to procure additional laboratory equipment nor is much time involved (only around 20 - 30 min for binding; Figure 3). For example, in a kind of field work, the inventive method can be even carried out using a syringe, for example by physician or even a veterinarian in a building for live stock.

The methods for the accumulation or concentration of prion proteins known from the prior art are usually limited as regards to target volumes. However, according to the present invention, target volumes and target solutions can be adjusted independently from the starting medium.

The present invention thus provides a reliable method that can easily be carried out and which provides a couple of benefits to amyloid protein tests known from the prior art.

According to the present invention, the terms "amyloid proteins" or "amyloids" , which can be used interchangeably herein, designate insoluble fibrous proteins aggregates sharing specific structural traits. The accumulation and/or abnormal deposition of such "amyloid proteins" leads to amyloidosis and/or disease conditions.

According to the present application, "amyloid proteins" or "amyloids" relate to a native and/or denatured protein form.

Thus, according to the invention amyloid proteins can be detected, isolated, purified and/or provided in a native and/or denatured form.

Preferably, the amyloid proteins are selected from beta amyloid (Aβ), which is, e.g., known from Alzheimer's disease, IAPP amylin (AIAPP), which is known, e.g., from diabetes mellitus type II, alpha-synuclein (SNCA), which is known from Parkinson's disease, Huntingtin, which is known from huntington's disease and in particular prion proteins (PrP), which are, e.g., known from prion diseases like BSE, CJD, CWD, and scrapie. PrP proteins represent an especially preferred embodiment of the present invention and comprise in particular PrP^{Sc}, PrP^{C}, PrPres and/or recombinant PrP proteins, fragments and/or derivatives thereof.

"Fragment and/or derivative" relates to any functionally active molecule showing characteristics corresponding to the characteristics of the corresponding "native amyloid protein", such as biologically activity and/or antigenic properties. For example, in terms of the invention, a fragment of a PrP protein shows antigenic properties corresponding to the PrP protein itself.

Step (a) contacting a sample with material suitable for carrying out IMAC is preferably carried out column chromatographically. However, carrying out step (a) is not bound to a particular method or technique. Suitable techniques for contacting comprise for example Fast Protein Liquid Chromatography (FPLC), high-pressure as well as low pressure systems, pump systems, syringes etc.

According to an especially preferred embodiment, the sample may be applied onto the material suitable for carrying out IMAC by centrifugation, preferably using a spin column. According to another especially preferred embodiment, the sample may be applied onto the material suitable for carrying out IMAC using variable pressure conditions at constant flow rates.

The carrier material is preferentially porous, e.g. carbohydrate based (e.g. Agarose, Sepharose) or a synthetic polymer such as methacrylate (e.g. Toyopearl, Fractogel, UNOsphere), or polystyrene (e.g. Source, Poros, Amberchrom), and is functionalized with chelating groups charged with metal ions (immobilized Metal Affinity Chromatography, IMAC) such as Al³⁺, Ca²⁺, CO²⁺, Cu²⁺, Cr³⁺, Fe³⁺, Ga³⁺, Mn²⁺, Ni²⁺, Zn²⁺, Yb³⁺.

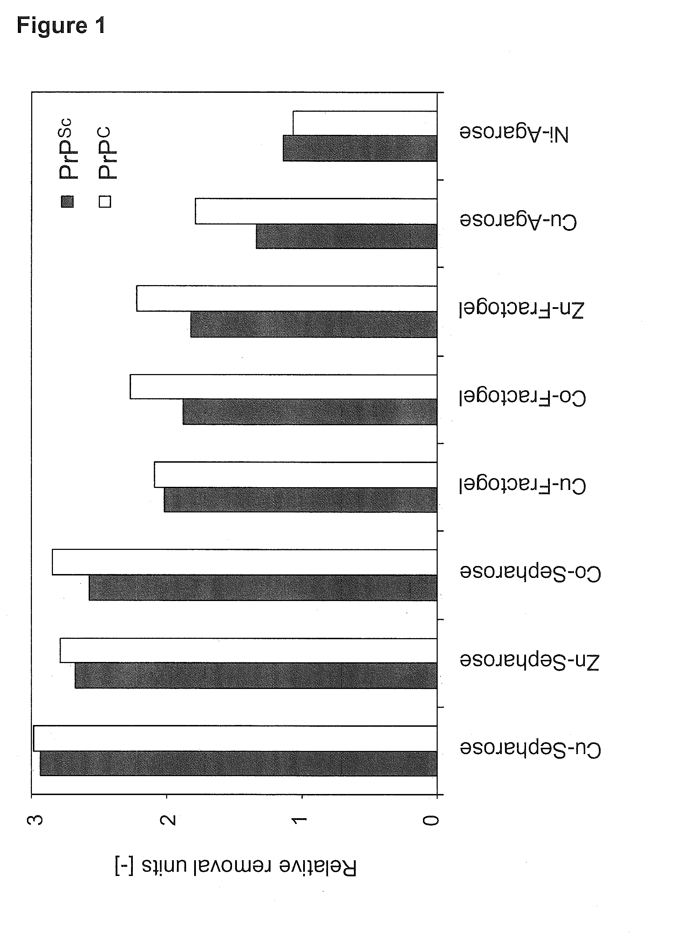

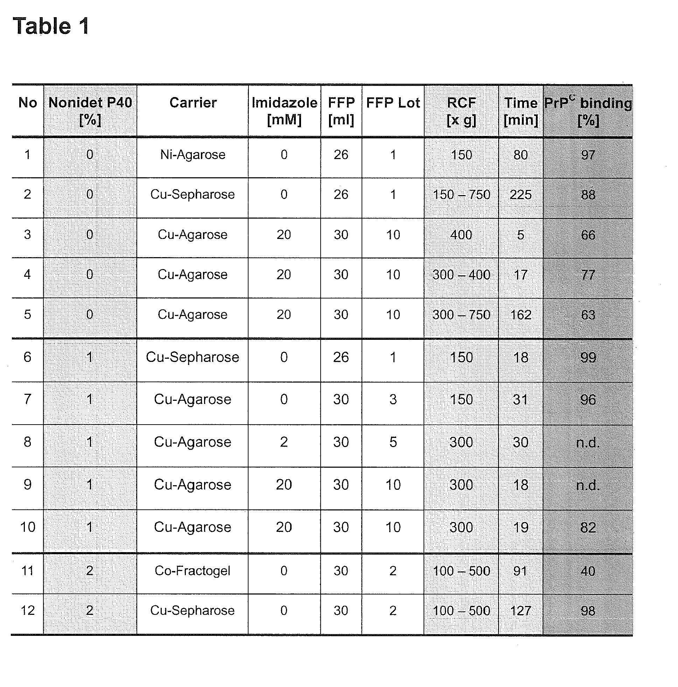

It has been found out, that PrP^{Sc} shows the highest affinity towards a given copper charged IMAC material (Figure 1). Thus, according to an especially preferred embodiment, Cu²⁺ is used, in particular in combination with a carbohydrate based matrix for diagnostic purposes. According to another especially preferred embodiment Zn²⁺ is preferred for removal purposes.

The sample which may be used according to the present invention may be any protein containing solution. It may be highly complex and protein rich, such as blood plasma, but also samples of lower complexity and/or low protein concentration may be used. The sample is preferably selected from blood, blood-derived compounds and compositions, serum, plasma, urine, saliva, milk, tears, semen, cerebrospinal fluid, ductal fluid. The sample may be a human sample but, of course, samples from animals are also comprised, in particular bovine samples and samples from sheep, elk and deer.

According to an especially preferred embodiment, the sample is plasma or plasma derived.

A further example for a sample according to the present invention is any intermediate product and/or solution present in pharmacological production processes.

A further example for a sample according to the present invention is any intermediate product and/or solution present in cosmetic and/or food production processes.

According to step (a) of the method of the present invention, detergents are added to the sample if it was not pretreated with detergents.

In terms of the present application, a detergent is a surfactant or a mixture of surfactants having "cleaning properties in dilute solutions." A detergent or surfactant lowers the surface tension of a liquid or the interfacial tension between two liquids. In general, each detergent known to the person skilled in the art may be used provided that amyloid protein binding to the IMAC material is not negatively influenced.

A detergent according to the present invention may be preferably selected from ethoxylates, cationic detergents, anionic detergents and/or non-ionic or zwitterionic detergents, wherein non-ionic detergents are especially preferred. Non-ionic detergents are characterized by their net uncharged, hydrophilic headgroups. For example, they may be based on polyoxyethylene glycol (e.g. Tween, Triton, Brij etc.), glycosides (e.g. maltosides, octyl-thioglcoside etc.), lipids (e.g. HEGAs), phosphine oxides, bile acids (e.g. DOC) and /or CHAPS.

According to an especially preferred embodiment, the present invention relates to nonionic detergents.

Dependent on the protein concentration of the sample to be treated the end concentration of the detergent in the sample is preferably from 0.01 to about 1.5 % (weight/volume; w/v), more preferably 0.2 to 1.2 % (w/v) and even more preferably about 0.8 to 1.2 % (w/v) and most preferably about 1.0 % (w/v).

In an particular preferred embodiment Triton X-100 and/or Nonidet P40 and/or IGEPAL CA-630 is used at a concentration of 1.0 % (w/v). It was found out that higher detergent concentrations, like 2 % (w/v), may have a detrimental effect on the retention times of the sample on the column, dependent on the matrix used (Table 1).

In a particularly preferred embodiment, in case of detergent untreated plasma, i.e. fresh frozen plasma, it was found out that the addition of nonionic detergents such as e.g. Nonidet P40, IGEPAL CA-630, or Triton X-100 (around 1% final concentration) facilitates handling and improves prion binding efficiency when using the carrier materials in spin columns:
- Higher prion binding yields and thus higher prion enrichment factors are observed than without detergent, enabling improved prion test sensitivities (Figure 2).
- Fresh frozen plasma without addition of these detergents tends to clog the spin columns at low centrifugal forces, or passes the spin columns at increased centrifugal forces faster than required for efficient prion binding, which necessitates manual adjustment of centrifugal forces during the process (Figure 3).

According to an especially preferred embodiment, samples comprise about 1% final concentration Nonidet P40.

Beside a treating of the sample with detergent, a sample used in step (a) of the present invention can be also pretreated by methods reducing the complexity of the sample like filtration steps, precipitation steps, addition of further "clearing agents" and/or digest of certain substances or proteins comprised in the sample. However, such additional steps are not necessary.

Further, agents may be added which avoid unspecific binding of non-target proteins on the IMAC material. Such agents are known to the person skilled in the art, such as imidazole. The imidazole concentration added depends on the complexity of the sample and may vary in a range of 0 mM to 50 mM, preferably 2 mM to 20 mM.

The flow through which is collected according to step (a) has mostly been purified from amyloids and proteins with the ability to become amyloidogenic, fragments and/or derivatives thereof, in particular prion proteins, or at least significantly purified. Thus, the flow- through, shows compared to the starting sample itself, a significantly lower risk of infection. Therefore, the flow-through provided may be used for medical purposes. For example, plasma must not be transferred to patients immediately. It has to be treated with a method for reducing the risk of infection beforehand. Such a method for amyloid removal and especially prion removal in addition to state of the art virus inactivation and/or removal is provided by the present invention.

According to a further preferred embodiment, the inventive method comprises step (b) optional removal of unbound substances. According to preferred embodiments, step (b) may be carried out by centrifugation or in a syringe. According to a further preferred embodiment, step (b) is carried out by means of a washing solution. Step (b) may be carried out once or several times, such as two times, three times etc.

The washing solution used in step (b) can be any buffer for purifying proteins which is known to a person skilled in the art and which does not affect binding of amyloids and proteins with the ability to become amyloidogenic to the IMAC material. According to an especially preferred embodiment, the buffer is a phosphate buffer, e.g. phosphate-buffered saline (PBS). Preferably, the buffer shows a pH value from about 4.0 to about 12.0, preferably about 7.0 to about 8.0 and most preferably about 7.2.

After step (b) the IMAC material comprising immobilized amyloids and proteins with the ability to become amyloidogenic may be directly used in further methods, such as Western Blots.

Of course, it is contemplated that after step (b) further steps such as step (c) elution amyloids and proteins with the ability to become amyloidogenic, fragments and/or derivatives thereof providing an eluate comprising amyloids and proteins with the ability to become amyloidogenic, fragments and/or derivatives thereof, and step (d) concentrating the eluate amyloids and proteins with the ability to become amyloidogenic, fragments and/or derivatives thereof to a desired volume can follow.

The elution step (c) is preferably carried out in salt solution. Of course, step (c) may be repeated. According to an especially preferred embodiment, the salt solution is aqueous NaCl and/or EDTA. The solution preferably has a pH value of about 4.0 to about 8.0, more preferably about 7.0. The NaCl concentration is preferably from about 0.1 M to about 0.8 M, more preferably about 0.3 M to about 0.7 M, and even more preferably about 0.5 M. The EDTA concentration is preferably from about 0.001 M to about 0.5 M, more preferably 0.1 M to about 0.3 M and even more preferably about 0.2 M.

Concentration step (d) may be carried out by any method for concentrating protein solution which is known to a person skilled in the art, such as filtration, dialysis, immunoprecipitation, etc. However, filtration, in particular centrifugal filtration, is preferred. Such filtration is preferably achieved by means of a centrifugal filter, which preferably has a molecular weight cut off of about smaller than 35 kDa, preferably about 1 kDa to about 15 kDa. According to an especially preferred embodiment, the molecular weight cut off is about 10 kDa for prions.

Another embodiment of the present invention comprises further a step for buffer exchange, which is carried out after step (d).

An especially preferred embodiment of the present invention relates to a method for isolating, concentrating and/or purifying prion proteins, fragments and/or derivatives thereof, comprising the steps:
(a) contacting a sample, in particular plasma, comprising prion proteins, fragments and/or derivatives thereof with a material suitable for carrying out immobilized metal affinity chromatography (IMAC)
wherein detergents are added to the sample if it was not pretreated with detergents.

One aspect of the present invention relates to a method for the removal of amyloids and proteins with the ability to become amyloidogenic. Hereby, the concentration of amyloids and proteins with the ability to become amyloidogenic, fragments and/or derivatives thereof of the flow-through is substantially decreased versus the original sample. This removal or depletion step is preferably carried out under constant flow conditions.

Another especially preferred embodiment of the present invention relates to a method for isolating, concentrating and/or purifying prion proteins, fragments and/or derivatives thereof, comprising the steps:
(a) contacting a sample, in particular plasma, comprising prion proteins, fragments and/or derivatives thereof with a material suitable for carrying out immobilized metal affinity chromatography (IMAC), and optionally
(b) optionally removing unbound substances, and optionally
(c) elution of prion proteins, fragments and/or derivatives thereof providing an eluate, and optionally
(d) concentrating the eluate comprising prion proteins to a desired volume,
wherein detergents are added to the sample if it was not pretreated with detergents.

Another aspect of the present invention relates to a method of in vitro diagnosis and/or quantification using a method according to the present invention comprising the final step of detecting amyloids and proteins with the ability to become amyloidogenic, a fragment and/or a derivative thereof.

This detection step is preferably carried out immunologically. For example,

Western blot analysis or enzyme-linked immunosorbent assay (ELISA) can be used to detect and quantify the presence of amyloid proteins in the sample. Of course, detection may also proceed by any other suitable method, known to the person skilled in the art such gel-mobility-shift assays, fluorescent in situ hybridization analysis, or other methods which track a protein upon alteration in size, charge and/or conformation. The particular method, label or detectable group used in the detection and/or quantification assay is not a critical aspect of the invention.

Of course, the method is particularly suitable for the detection and the diagnosis of amyloid related diseases such as Alzheimer's disease (related to the detection of beta amyloid), Parkinson's disease (alpha-synuclein), diabetes mellitus type 2 (IAPP amylin), and prion protein related diseases such as BSE, CJD, CWD, scrapie, etc. Of course, the method of the invention can be combined with methods or tests which are known for the detection of the specified diseases.

### BRIEF DESCRIPTION OF THE FIGURES AND TABLES

Figure 1 is a graphic representation of the binding or removal of PrP^{Sc} and PrP^{C} using IMAC beads. Cu-Sepharose: Cu²⁺ charged IMAC Sepharose High Performance beads (Ge-Healthcare); Zn-Sepharose: Zn²⁺ charged IMAC Sepharose High Performance beads (Ge-Healthcare); Co-Sepharose: Co²⁺ charged IMAC Sepharose High Performance beads (Ge-Healthcare); Cu-Fractogel: Fractogel EMD IMAC (M) beads (Merck) charged with Cu²⁺; Co-Fractogel: Fractogel EMD IMAC (M) beads (Merck) charged with Co²⁺; Zn-Fractogel: Fractogel EMD IMAC (M) beads (Merck) charged with Zn²⁺; Cu-Agarose: IMAC agarose beads (Sartorius) charged with Cu²⁺; Ni-Agarose: IMAC agarose beads (Sartorius) charged with Ni²⁺. Materials and methods are as indicated in Example 1.

Table 1 shows the binding or removal of prions present in fresh frozen plasma using IMAC spin columns, and the influence of Nonidet P40 on centrifugation performance. No: Number for identification; Nonidet P40 [mM]: Final concentration of added Nonidet P40; Carrier: see Figure 1 description for details; Imidazole [mM]: Final concentration of added imidazole; FFP: Fresh frozen plasma; FFP lot: Internal lot number of used FFP; RCF [x g]: Relative centrifugal force applied to pass FFP through the spin column. In some cases RCF ranges are indicated, meaning that the RCF had to be increased successively from the lower value to the upper value in order to pass FFP through the spin column; Time [min]: Total time to pass the indicated FFP volume through the spin column; PrP^{C} binding [%]: % of initial PrP^{C} in plasma that was removed or bound by the spin column procedure. See Example 2 for details regarding materials and methods.

Figure 2 is a graphic representation of the binding or removal of prions present in 30 ml of fresh frozen plasma (FFP) or a detergent pretreated plasma (Octaplas S/D), both supplemented with 20 mM imidazole, using IMAC spin columns. X-Axis indicates the final concentration of added detergent. Unless specified in brackets, the relative centrifugation force (RCF) applied was 300 x g. In some cases RCF ranges are indicated, meaning that the RCF had to be increased successively from the lower value to the upper value in order to pass the plasma through the spin column. See Example 3 for details regarding materials and methods.

Figure 3 is a graphic representation of the centrifugation time or pre-analysis time required to process 30 ml of fresh frozen plasma (FFP) or a detergent pretreated plasma (Octaplas S/D), both supplemented with 20 mM imidazole, using IMAC spin columns. X-Axis indicates the final concentration of added detergent. Unless specified in brackets, the relative centrifugation force (RCF) applied was 300 x g. In some cases RCF ranges are indicated, meaning that the RCF had to be increased successively from the lower value to the upper value in order to pass the plasma through the spin column. See Example 3 for details regarding materials and methods.

Table 2 summarizes a purification and concentration process of plasma prions comprising the steps (a) contacting human plasma with IMAC beads by means of a spin column, (b) removing unbound substances, (c) elution of PrP^{C}, (d) concentrating the obtained eluate and exchanging the elution buffer with phosphate buffered saline (PBS). See Example 4 for details regarding materials and methods.

### Examples

### Example 1: Binding or removal of PrP^{Sc} and PrP^{C} using IMAC beads

Deionized and filtered water was used for the preparation of solutions.

### Preparation:

All IMAC beads were modified based on standard procedures (see, e.g., GE-Healthcare or Merck product information sheets). All solutions were filtered prior to use (Whatman cellulose acetate membrane filters with 0.45 µm pore size).

Ni-Agarose: IMAC agarose beads charged with Ni²⁺ were taken from the commercially available Sartorius VS-MCMAXIB spin columns.

Cu-Agarose: IMAC agarose beads (Sartorius) charged with Cu²⁺ were prepared using the spin column procedure described in Example 2.

Co-Fractogel: The preparation of nude Fractogel EMD IMAC (M) beads is described in Example 2. After re-suspension and filtration with water (Whatman cellulose acetate membrane filter with 0.45 µm pore size), 5 g (wet weight) of the bead pellet was transferred into a closable 50 ml plastic tube. The bead pellet was dissolved in 45 ml of 0.25 M CoCl₂ and placed on an overhead shaker for 30 min (RT, 5 rpm). The metal charging solution was removed by filtration. The bead pellet was again dissolved in 45 ml of 0.25 M CoCl₂ and placed on an overhead shaker for 30 min (RT, 5 rpm), following removal of the metal charging solution by filtration. The obtained bead pellet was filtered and washed twice with 100 ml of NaCl (0.5 M). Finally, the beads were re-suspended and filtered three times with 100 ml of phosphate buffered saline (PBS) pH 7.2.

Cu-Fractogel: The preparation of nude Fractogel EMD IMAC (M) beads is described in Example 2. After re-suspension and filtration with water (Whatman cellulose acetate membrane filter with 0.45 µm pore size), 5 g (wet weight) of the bead pellet was transferred into a closable 50 ml plastic tube. The bead pellet was dissolved in 45 ml of 0.25 M CuSO₄ and placed on an overhead shaker for 30 min (RT, 5 rpm). The metal charging solution was removed by filtration. The bead pellet was again dissolved in 45 ml of 0.25 M CuSO₄ and placed on an overhead shaker for 30 min (RT, 5 rpm), following removal of the metal charging solution by filtration. The obtained bead pellet was filtered and washed twice with 100 ml of NaCl (0.5 M). Finally, the beads were re-suspended and filtered three times with 100 ml of PBS (pH 7.2). Zn-Fractogel: The preparation of nude Fractogel EMD IMAC (M) beads is described in Example 2. After re-suspension and filtration with water (Whatman cellulose acetate membrane filter with 0.45 µm pore size), 5 g (wet weight) of the bead pellet was transferred into a closable 50 ml plastic tube. The bead pellet was dissolved in 45 ml of 0.25 M ZnCl₂ and placed on an overhead shaker for 30 min (RT, 5 rpm). The metal charging solution was removed by filtration. The bead pellet was again dissolved in 45 ml of 0.25 M ZnCl₂ and placed on an overhead shaker for 30 min (RT, 5 rpm), following removal of the metal charging solution by filtration. The obtained bead pellet was filtered and washed twice with 100 ml of NaCl (0.5 M). Finally, the beads were re-suspended and filtered three times with 100 ml of PBS (pH 7.2). Co-Sepharose, Cu-Sepharose, and Zn-Sepharose beads were prepared from nude IMAC Sepharose High Performance beads (GE-Healthcare) according to the suppliers instructions. The metal-ion charged and washed beads were re-suspended and filtered three times with 100 ml of PBS (pH 7.2).

### Binding or removal of PrP^{Sc} and PrP^{C} using IMAC spin columns:

PBS (pH 7.2) was filtered prior to use (Whatman cellulose acetate membrane filters with 0.45 µm pore size). Preparation of the beads for the binding or removal of PrP^{Sc} and PrP^{C}: The beads were re-suspended and filtered three times with PBS (pH 7.2). 1 ml of the solution containing PrP^{Sc} and PrP^{C} consisted of 20 µl of a 10 % scrapie brain homogenate (prepared using hamster brains infected with the 263K hamster-adapted agent) and 980 µL PBS (pH 7.2). 1 ml of this solution was added to 0.1 g (wet weight) of bead pellet in a 1.5 ml plastic tube. The tube was closed and the mixture was incubated for 2 h on an overhead shaker (4 °C, 20 rpm).

Control samples: the same solution containing PrP^{Sc} and PrP^{C} was added into an empty 1.5 ml plastic tube (without beads). The tube was closed and incubated for 2 h on an overhead shaker (4 °C, 20 rpm). In addition, 20 µl of a 10 % non-infected hamster brain homogenate was added to 980 µL PBS (pH 7.2) in a 1.5 ml plastic tube (without beads). The tube was closed and incubated for 2 h on an overhead shaker (4 °C, 20 rpm).

After incubation, supernatants (free of any residual beads) were transferred into a new plastic tube and analyzed directly.

### Analysis:

A conformation-dependent immunoassay (CDI) was applied to differentiate between total PrP and PrP^{C} concentrations. 20 µl of supernatant solution was either supplemented with 20 µl PBS or 20 µl guanidine hydrochloride solution (8 M). The samples were incubated for 5 min at 80 °C (closed 1.5 ml plastic tubes), diluted with HPE (High Performance ELISA) dilution buffer (Sanquin), and analyzed using a sandwich enzyme-linked immunosorbent assay (ELISA). For sandwich ELISA, white 96 well flat-bottom plates (Nunc MaxiSorp) were prepared with 3F4 as a capture antibody (5 µg/ml in carbonate-bicarbonate buffer pH 9.4), and blocked with SuperBlock Blocking Buffer (Thermo Scientific). Plasma samples were diluted in HPE (High Performance ELISA) dilution buffer (Sanquin) and applied to the plate. Biotinylated SAF32 antibody (135 ng/ml in HPE) was added, following streptavidin-poly-HRP (Thermo Scientific; in HPE), and luminescent detection using SuperSignal Femto solution (Thermo Scientific). Plate washing between the different steps, except after blocking, was carried out with tris buffered saline (TBS; pH 7.2) supplemented with 0.05 % Tween-20. PrP^{C} and PrP^{Sc} binding or removal was quantified by comparing concentration differences with the control samples.

### Results:

IMAC beads are able to bind or remove PrP^{Sc} and PrP^{C} (Figure 1).

### Example 2: Binding or removal of prions present in fresh frozen plasma using IMAC spin columns, and influence of Nonidet P40 on centrifugation performance

Commercially available Sartorius VS-MCMAXIB spin columns filled with Ni²⁺ charged agarose were either used directly, or modified as described in the following. Deionized and filtered water was used for the preparation of solutions.

### Preparation:

Modifications were carried out at in a centrifuge (150 x g, 25 °C) equipped with a swing-bucket rotor, and using standard protocols (see, e.g., GE-Healthcare or Merck product information sheets). All solutions were filtered prior to use (Whatman cellulose acetate membrane filters with 0.45 µm pore size).

Cu-Agarose: The spin column was assembled as recommended by the supplier. The storage medium was removed with three successive portions of 11 ml of water. Ni²⁺ was removed from the beads with three successive portions of 11 ml of a solution containing 0.5 M NaCl, 0.2 M EDTA (pH 7). Subsequently, the beads were washed with three successive portions of 11 ml of 0.5 M NaCl. The washed beads were recharged with three successive portions of 10 ml of 0.25 M CuSO₄. Unbound CuSO₄ was removed with three successive portions of 11 ml of 0.5 M NaCl. Finally, three successive portions of 10 ml of phosphate buffered saline (PBS; pH 7.2) were applied to remove residual NaCl.

Cu-Sepharose: The Ni²⁺ charged agarose supplied with Sartorius VS-MCMAXIB plugs was replaced with the same volume (1.6 ml) of nude IMAC Sepharose High Performance beads (GE Healthcare), following assembly of the spin column as recommended by the supplier. Three successive portions of 11 ml of water were used to wash the beads. Subsequently, the beads were charged with three successive portions of 10 ml of 0.25 M CuSO₄. Unbound CuSO₄ was removed with three successive portions of 11 ml of 0.5 M NaCl. Finally, three successive portions of 10 ml of PBS (pH 7.2) were applied to remove residual NaCl.

Co-Fractogel: 4 g (dry weight) of Fractogel EMD Epoxy (M) beads (Merck) were dissolved in 50 ml of water. The water was removed by filtration (Whatman cellulose acetate membrane filter with 0.45 µm pore size). The bead pellet was re-suspended and filtered twice with 50 ml water. The bead pellet was transferred into a 50 ml plastic tube. Subsequently, 45 ml of iminodiacetic acid solution (2 M in 0.1 M sodium tetraborate, pH 9.3) was added and the tube was closed. The mixture was incubated for 90 h on an overhead shaker (RT, 5 rpm). Afterwards the iminodiacetic acid solution was removed by filtration. The obtained pellet of nude Fractogel EMD IMAC (M) beads was re-suspended and filtered three times with 150 ml water. In a subsequent step, the Ni²⁺ charged agarose supplied with Sartorius VS-MCMAXIB plugs was replaced with the same volume (1.6 ml) of the prepared nude Fractogel EMD IMAC (M) beads, following assembly of the spin column as recommended by the supplier. Three successive portions of 11 ml of water were used to wash the beads. Subsequently, the beads were charged with three successive portions of 10 ml of 0.25 M CoCl₂. Unbound CoCl₂ was removed with three successive portions of 11 ml of 0.5 M NaCl. Finally, three successive portions of 10 ml of PBS (pH 7.2) were applied to remove residual NaCl.

### PrP binding or removal using IMAC spin columns:

Solutions consisting of imidazole and/or PBS were filtered prior to use (Whatman cellulose acetate membrane filters with 0.45 µm pore size).

Fresh frozen plasma (from human) was defrosted in a water bath (35 °C, 25 min) and incubated for 10 min at RT. Optionally imidazole (1.3 M, pH 7.2) was added to a final concentration of 2 mM or 20 mM. Optionally Nonidet P40 (20 % w/v) was added to a final concentration of 1 % or 2 %. Optionally scrapie brain homogenate (prepared using hamster brains infected with the 263K hamster-adapted agent) was added to a final PrP^{Sc} concentration of up to 10 ng/ml. The scrapie brain homogenate was highly clarified prior to use by centrifugation at 4500 x g for 30 min (4 °C), following filtration with 0.45 µm centrifugal filters (10'000 x g, 4 °C), and filtration with 0.2 µm centrifugal filters (10'000 x g, 4 °C). Volume changes due to the addition of imidazole or Nonidet P40 were compensated with the corresponding identical volume of deionized water in fresh frozen plasma samples that were not supplemented with these substances. The final plasma volumes were 25 to 30 ml (see Table 1).

The spin columns were prepared as follows: centrifugation (150 x g, swing-bucket rotor, 20 °C) with three successive portions of 10 ml of PBS pH 7.2, following centrifugation (150 x g, swing-bucket rotor, 20 °C) with three successive portions of 10 ml of PBS pH 7.2 (for plasma without imidazole) or 2 mM imidazole in PBS pH 7.2 (for plasma containing 2 mM imidazole) or 20 mM imidazole in PBS pH 7.2 (for plasma containing 20 mM imidazole). After this pretreatment the spin columns were free of any residual liquid, except for the bead void volume.

Only plasma with a clear appearance (without precipitations, after complete dissolving of optionally added supplements) was applied to the spin columns. 30 ml Plasma samples: the plasma was applied in three successive portions of 10 ml to the pretreated spin columns (20 °C, swing-bucket rotor) at the centrifugal forces indicated in Table 1. 26 ml Plasma samples: the plasma was applied in two successive portions of 13 ml to the pretreated spin columns (20 °C, swing-bucket rotor) at the centrifugal forces indicated in Table 1.

### Analysis:

The PrP^{C} concentration before centrifugation and after passage through the spin columns was determined using a sandwich enzyme-linked immunosorbent assay (ELISA) and expressed as binding yield (% of initial PrP^{C} in plasma that was removed by the spin column procedure). For sandwich ELISA, white 96 well flat-bottom plates (Nunc MaxiSorp) were prepared with 3F4 as a capture antibody (5 µg/ml in carbonate-bicarbonate buffer pH 9.4), and blocked with SuperBlock Blocking Buffer (Thermo Scientific). Plasma samples were diluted in HPE (High Performance ELISA) dilution buffer (Sanquin) and applied to the plate. Biotinylated SAF32 antibody (270 ng/ml in HPE) was added, following streptavidin-poly-HRP (Thermo Scientific; in HPE), and luminescent detection using SuperSignal Femto solution (Thermo Scientific). Plate washing between the different steps, except after blocking, was carried out with tris buffered saline (TBS; pH 7.2) supplemented with 0.05 % Tween-20.

### Results:

PrP^{Sc} and PrP^{C} binding to IMAC beads in phosphate buffered saline is demonstrated in Example 1.

Using fresh frozen plasma, we experienced severe technical difficulties with the corresponding IMAC spin column chromatography process. IMAC spin columns tend to clog after passage of several column volumes of fresh frozen plasma. In this case manual adjustment of the centrifugation process is necessary to sustain plasma flow through the IMAC spin column (Table 1; No 2, 4 & 5). At the other hand, fresh frozen plasma tends to pass through spin columns too fast at increased centrifugal force (Table 1; No 3), which apparently reduces prion binding yield due to the lower contact time with the metal ligands. In overall, IMAC spin column chromatography with fresh frozen plasma is difficult to control, requires manual adjustment, and tends to bind less prions than desired (Table 1; No 1 - 5). Such a system is therefore not suitable for the binding or enrichment of prions from large volumes of fresh frozen plasma, especially if samples are processed in parallel, and if high enrichment factors are required to achieve high test-sensitivities after elution.

Surprisingly it was found out that these drawbacks can be solved by the addition of around 1 % of Nonidet P40 (final concentration). Clogging events are not observed and the centrifugation process does not require manual adjustment anymore (fixed centrifugation forces can be applied). Technical failures due to increased centrifugal force (e.g. spin column breakage) can thus be minimized, thereby improving process safety when handling infectious material. Furthermore, several column volumes of fresh frozen plasma can be passed through IMAC spin columns at comparably constant flow rates, which is commonly not observed in the absence of the detergent. Typically recommended chromatographic flow rates, allowing for high prion binding yields, are thus achieved (Table 1; 6 - 10 vs. 1 - 5). For example with Cu-Sepharose, PrP^{C} removal with Nonidet P40 (1 % final concentration) was 2 log₁₀ in 18 min (constant 150 x g), whereas PrP^{C} removal without Nonidet P40 (1 % final concentration) was only 0.9 log₁₀ in 225 min (manual adjustment 150 - 750 x g) (Table 1; 6 vs. 2).
2 % of Nonidet P40 (final concentration) was also added to fresh frozen plasma and applied to IMAC spin column chromatography (Table 1; 11 & 12). Although prion binding yields were not affected adversely, flow-through times or pre-analysis times were considerably longer than with 1 % Nonidet P40.

In overall, addition of around 1 % Nonidet P40 (final concentration) to fresh frozen plasma strongly improves handling, controllability, and prion binding yield during IMAC spin column chromatography. These advantages can be used for the binding or concentration of prions from large volumes of fresh frozen plasma, especially if samples are processed in parallel, and if high prion enrichment factors are required to achieve high test-sensitivities.

### Example 3: Binding or removal of prions present in 30 ml of fresh frozen plasma or detergent pretreated plasma using IMAC spin columns, and influence of added detergents on pre-analysis time

Deionized and filtered water was used for the preparation of solutions. Solutions consisting of imidazole and/or PBS were filtered prior to use (Whatman cellulose acetate membrane filters with 0.45 µm pore size).

### Preparation:

Commercially available Sartorius VS-MCMAXIB spin columns filled with Ni²⁺ charged agarose were recharged with Cu²⁺ as described in Example 2 (Cu-Agarose).

Fresh frozen plasma (FFP) from human or Octaplas S/D was defrosted in a water bath (35 °C, 25 min) and incubated for 10 min at RT. Imidazole (1.3 M in H₂O, pH 7.2) was added to a final concentration of 20 mM. Optionally Nonidet P40, IGEPAL CA-630, Triton X-100, ZW 3-12 (N-Dodecyl-N,N-dimethyl-3-ammonio-1-propanesulfonate), or SDS (all 20 % w/v in water) was added to a final concentration of 1 %. Plasma without added detergent was supplemented with the corresponding identical volume of water. Accordingly, the final plasma volumes were always 30 ml. All plasma solutions had a clear appearance (without precipitations), and the detergents were completely dissolved.

### Binding or removal of plasma prions using IMAC spin columns:

The spin columns were prepared as follows: centrifugation (150 x g, swing-bucket rotor, 20 °C) with three successive portions of 10 ml of 20 mM imidazole in PBS (pH 7.2). After this pretreatment the spin columns were free of any residual liquid, except for the bead void volume.

The plasma solutions were applied in three successive portions of 10 ml to the pretreated spin columns (20 °C, swing-bucket rotor) at 300 x g unless stated otherwise in Figure 2 and Figure 3 (X-axis).

Note: for some plasma solutions, centrifugal forces had to be successively increased to pass the plasma through the spin columns. In this case, lowest and the highest RCF values are indicated in brackets in Figure 2 and Figure 3 (X-axis).

### Analysis:

The PrP^{C} concentration before centrifugation and after passage through the spin columns was determined using a sandwich enzyme-linked immunosorbent assay (ELISA) and expressed as binding yield (% of initial PrP^{C} in plasma that was removed by the spin column procedure). For sandwich ELISA, white 96 well flat-bottom plates (Nunc MaxiSorp) were prepared with 3F4 as a capture antibody (5 µg/ml in carbonate-bicarbonate buffer pH 9.4), and blocked with SuperBlock Blocking Buffer (Thermo Scientific). Plasma samples were diluted in HPE (High Performance ELISA) dilution buffer (Sanquin) and applied to the plate. Biotinylated SAF32 antibody (270 ng/ml in HPE) was added, following streptavidin-poly-HRP (Thermo Scientific; in HPE), and luminescent detection using SuperSignal Femto solution (Thermo Scientific). Plate washing between the different steps, except after blocking, was carried out with tris buffered saline (TBS; pH 7.2) supplemented with 0.05 % Tween-20.

### Results:

Fresh frozen plasma supplemented with non-ionic detergents such as Nonidet P40, IGEPAL CA-630, or Triton X-100 (around 1 % final concentration) shows the same advantages over fresh frozen plasma without detergent during IMAC spin column chromatography as disclosed in Example 2:

Addition of around 1 % Nonidet P40, IGEPAL CA-630, or Triton X-100 (final concentration) to fresh frozen plasma strongly improves handling, controllability, and prion binding yield during IMAC spin column chromatography (Figure 2 and Figure 3). These advantages can be used for the binding or concentration of prions from large volumes of fresh frozen plasma, especially if samples are processed in parallel, and if high prion enrichment factors are required to achieve high test-sensitivities.

The production of Octaplas S/D consists of a detergent treatment comprising the steps (i) addition of 1 % Trition X-100 (final concentration) to fresh frozen plasma, (ii) incubation for some time, (iii) removal of Trition X-100. This detergent "pretreatment" leads to the same advantages (facilitated handling, better process controllability, higher prion binding yield), such as when the detergent is added to fresh frozen plasma and present during IMAC spin column chromatography (Figure 2 and Figure 3). Further addition of the same detergent (or Nonidet P40, or IGEPAL CA-630) at the same concentration to Octaplas S/D did not result in any further diagnostic advantage (e.g. prion binding yield; Figure 2) or technical advantage (e.g. clogging; Figure 3) using IMAC spin column chromatography.

Addition of the anionic detergent SDS or zwitterionic detergent ZW 3-12 (around 1 % final concentration each) to fresh frozen plasma or Octaplas S/D strongly reduced prion binding yields (Figure 2). Since high test-sensitivities require high enrichment factors, these detergents are not recommended with IMAC based enrichments.

Octaplas S/D shows generally lower PrP binding yields than fresh frozen plasma (Figure 2). This might correlate with the PrP^{C} concentration in Octaplas S/D, which is approximately 15 % lower than in the used fresh frozen plasma.

In overall, addition of non-ionic detergents such as Nonidet P40, IGEPAL CA-630, or Triton X-100 (around 1 % final concentration) to fresh frozen plasma strongly improves handling, controllability, and prion binding yields during IMAC spin column chromatography. Similar advantages are observed if these detergents are removed prior to IMAC spin column chromatography, as shown for Octaplas S/D. The method presented here can thus be used for the parallel processing of multiple samples of fresh frozen plasma and pretreated plasma such as Octaplas S/D.

### Example 4: Purification and concentration of plasma prions

Deionized and filtered water was used for the preparation of solutions. Solutions consisting of PBS or NaCl or EDTA were filtered prior to use (Whatman cellulose acetate membrane filters with 0.45 µm pore size).

### Preparation:

Commercially available Sartorius VS-MCMAXIB spin columns filled with Ni²⁺ charged agarose were recharged with Cu²⁺ as described in Example 2 (Cu-Agarose). The spin columns were prepared as follows: centrifugation (150 x g, swing-bucket rotor, 20 °C) with three successive portions of 10 ml of PBS pH 7.2. After this pretreatment the spin columns were free of any residual liquid, except for the bead void volume.

Fresh frozen plasma from human was defrosted in a water bath (35 °C, 25 min) and incubated for 10 min at RT. Nonidet P40 (20 % w/v) was added to a final concentration of 1 %. Only plasma with a clear appearance (without precipitations, after complete dissolving of detergent) was used in the following procedure.

### (a) Contacting human plasma with IMAC beads:

The plasma was applied in three successive portions of 10 ml to a pretreated spin column in a centrifuge (20 °C, 150 x g, swing-bucket rotor).

### (b) Removing unbound substances:

Subsequently, after step (a), PBS (pH 7.2) was applied in three successive portions of 10 ml to the spin column in a centrifuge (20 °C, 150 x g, swing-bucket rotor).

### (c) Elution of prions:

Subsequently, after step (b), a solution consisting of 0.5 M NaCl and 0.2 M EDTA (pH 7.2) was applied in five successive portions of 10 ml to the spin column in a centrifuge (20 °C, 150 x g, swing-bucket rotor).

### (d) Eluate concentration and buffer exchange:

Subsequently, after step (c), the obtained eluate was concentrated using 10 kDa Amicon Ultra-15 Centrifugal Filter Units in a centrifuge (20 °C, 3900 x g, swing-bucket rotor). The obtained retentate (1.5 ml) was diluted twice with PBS pH 7.2 (around 13 ml) in the same filter unit and concentrated (20 °C, 3900 x g, swing-bucket rotor).

### Analysis:

PrP^{C} enrichment factor was determined experimentally by comparing the PrP^{C} concentration in plasma (before steps a - d) with the PrP^{C} concentration after purification and concentration (after steps a - d) using a sandwich enzyme-linked immunosorbent assay (ELISA). PrP^{C} yield [%] was calculated from the PrP^{C} enrichment factor and the maximal enrichment factor possible (volume ratio of plasma and concentrate).

For the sandwich ELISA, white 96 well flat-bottom plates (Nunc MaxiSorp) were prepared with 3F4 as a capture antibody (5 µg/ml in carbonate-bicarbonate buffer pH 9.4), and blocked with SuperBlock Blocking Buffer (Thermo Scientific). Plasma samples were diluted in HPE (High Performance ELISA) dilution buffer (Sanquin) and applied to the plate. Biotinylated SAF32 antibody (270 ng/ml in HPE) was added, following streptavidin-poly-HRP (Thermo Scientific; in HPE), and luminescent detection using SuperSignal Femto solution (Thermo Scientific). Plate washing between the different steps, except after blocking, was carried out with tris buffered saline (TBS; pH 7.2) supplemented with 0.05 % Tween-20.

### Results:

Prions can be purified and concentrated (enriched) from complex mixtures such as human plasma into a target solution. The high prion yields achieved with large plasma volumes enable high prion enrichment factors. Using the method of the present invention it is thus possible to significantly improve the detection limit of low concentrations of prions in complex solutions (Table 2).

### [1] COMMISSION REGULATION (EC) No 21/2008 of 11 January 2008:

*"For the purposes of carrying out the rapid tests in accordance with Articles 5(3) and 6(1), the following methods shall be used* as *rapid tests for the monitoring of BSE in bovine animals:*
- *immuno-blotting test based on a Western blotting procedure for the detection of the Proteinase K resistant fragment PrPRes (Prionics-Check Western test),*
- *chemiluminescent ELISA test involving an extraction procedure and an ELISA technique, using an enhanced chemiluminescent reagent (Enfer test & Enfer TSE Kit version 2.0, automated sample preparation),*
- *microplate based immunoassay for the detection of PrPSc (Enfer TSE Version 3),*
- *sandwich immunoassay for PrPRes carried out following denaturation and concentration steps (Bio-Rad Te-SeE test),*
- *microplate based immunoassay (ELISA) which detects Proteinase K resistant PrPRes with monoclonal antibodies (Prionics-Check LIA test),*
- *conformation-dependent immunoassay, BSE antigen test kit (Beckman Coulter InPro CDI kit), chemiluminescent ELISA for qualitative determination of PrPSc (CediTect BSE test),*
- *immunoassay using a chemical polymer for selective PrPSc capture and a monoclonal detection antibody directed against conserved regions of the PrP molecule (IDEXX HerdChek BSE Antigen Test Kit, EIA),*
- *microplate based chemiluminescent immunoassay for the* d*etection of PrPSc in bovine tissues (Institut Pourquier Speed'it BSE),*
- *lateral flow immunoassay using two different monoclonal antibodies to detect Proteinase K resistant PrP fractions (Prionics Check PrioS-TRIP),*
- *two-sided immunoassay using two different monoclonal antibodies directed against two epitopes presented in a highly unfolded state of bovine PrPSc (Roboscreen Beta Prion BSE EIA Test Kit),*
- *sandwich ELISA for the detection of Proteinase K resistant PrPSc (Roche Applied Science PrionScreen),*
- *antigen-capture ELISA using two different monocloncal antibodies to detect Proteinase K resistant PrP fractions (Fujirebio FRELISA BSE post-mortem rapid BSE Test).* "

## Claims

1. Method for isolating, concentrating and/or purifying amyloids and/or proteins with the ability to become amyloidogenic, fragments and/or derivatives thereof, comprising a step
(a) contacting a sample comprising amyloids and/or proteins with the ability to become amyloidogenic, fragments and/or derivatives thereof with a material suitable for carrying out immobilized metal affinity chromatography (IMAC), wherein detergents are added to the sample if it was not pretreated with detergents.

2. Method according to claim 1, further comprising the step
(b) optionally removing unbound substances
(c) elution of amyloids and/or proteins with the ability to become amyloidogenic, fragments and/or derivatives thereof providing an eluate comprising the amyloids and/or proteins with the ability to become amyloidogenic, and
(d) concentrating the eluate comprising the amyloid proteins to a desired volume.

3. Method according to claim 1 or 2, wherein the amyloids and/or proteins with the ability to become amyloidogenic, fragments and/or derivatives thereof are selected from beta amyloid, IAPP amylin, alpha-synuclein and PrP protein, in particular PrP^{Sc}, PrP^{C}, PrPres and/or recombinant prion protein.

4. Method according to claim 1, wherein step (a) is carried out chromatographically, preferably by centrifugation and more preferably by centrifugation using a spin column.

5. Method according to claim 1 or 2, wherein the material suitable for carrying out IMAC comprises a matrix with attached chelating groups that are charged with metal ions such as Al³⁺, Ca²⁺, Co²⁺, Cu²⁺, Cr³⁺, Fe³⁺, Ga³⁺, Mn²⁺, Ni²⁺, Zn²⁺, Yb³⁺.

6. Method according to any one of claims 1-4, wherein the sample is selected from blood, blood derived compounds and compositions, serum, plasma, urine, saliva, milk, tears, semen, cerebrospinal fluid, and/or ductal fluid and is preferably plasma.

7. Method according to any one of claims 1-6, wherein the detergent is preferably a detergent, more preferably a non-ionic detergent, most preferably Triton X-1 00 and/or Nonidet P40 and/or IGEPAL CA-630.

8. Method according to any one of claims 1-7, wherein step (b) is carried out by means of a washing solution, preferably in several washing steps, preferably by centrifugation.

9. Method according to claim 8, wherein the washing solution, preferably PBS, has a pH value from about 4.0 to about 12.0, preferably about 7.0 to about 8.0, most preferably about 7.2.

10. Method according to claims 1-9, wherein the elution in step (c) is carried out in salt solution and is preferably repeated.

11. Method according to claim 10, wherein the salt solution, which is preferably aqueous NaCl and/or EDTA, has a pH value of about 4.0 to about 8.0, more preferably about 7.0, wherein the NaCl concentration is preferably from about 0.1 M to about 0.8 M, more preferably is about 0.5 M, and wherein the EDTA concentration is preferably from about 0.001 M to about 0.5 M, and more preferably is about 0.2 M.

12. Method according to any one of claims 1-11, wherein the concentration of step (d) is carried out via filtration, preferably by means of a centrifugal filter that preferably has a molecular weight cut off of about smaller than 35 kDa, preferably about 1 kDa to about 15 kDa.

13. Method according to any one of claims 1-12, comprising further a step for buffer exchange which is carried out after step (d).

14. Method of in vitro diagnosis and/or quantification using a method according to any one of claims 1-13, comprising a final step for detecting amyloids and proteins with the ability to become amyloidogenic, a fragment and/or derivative thereof.

15. Method of diagnosis according to claim 14, wherein the detection step is carried out immunologically and/or by mass spectrometry.
